# EUROPEAN PATENT APPLICATION

(11) **EP 2 835 136 A1**
(43) Date of publication of application: **11.02.2015**
(21) Application number: 13179533.8
(22) Date of filing: 07.08.2013
(51) Int. Cl.: A61K 38/31, A61P 15/08

(54) **Somatostatin Receptor Modulator for the treatment of infertility**

(71) Applicant: PregLem S.A., 1228 Plan-les-Ouates Geneva (CH)
(72) Inventor: Tran, Thien Duc, 01630 Challex (FR); Gotteland, Jean-Pierre, 1205 Geneva (CH)
(74) Representative: Gullerné Lados, Zsuzsanna

(57) **Abstract**

The present invention relates to the acceleration of the start of growth of the quiescent follicles and/or to slowing down the depletion of the ovarian reserve by using a peptide having dual somatostatin antagonist/agonist activity, in order to treat infertility. The invention also relates to pharmaceutical composition and kit containing said peptide, and treatment methods, particularly treatment of infertility by using said peptide.

## Description

### FIELD OF THE INVENTION

The present invention relates to the acceleration of the start of growth of the quiescent follicles and/or to slowing down the depletion of the ovarian reserve by using a peptide having dual somatostatin antagonist/agonist activity, in order to treat infertility. The invention also relates to pharmaceutical composition and kit containing said peptide, and treatment methods, particularly treatment of infertility by using said peptide.

### BACKGROUND OF THE INVENTION

In women, as in all mammals, fertility is dependent on the presence in the ovaries of female gametes called "oocytes". In humans, the oocyte capital is constituted once and for all at birth; the number of oocytes is then comprised between 500 000 and 1 million per ovary. These oocytes are surrounded by a few granulosa cells; this functional group is called an ovarian follicle (Gougeon, A, Endocrine Reviews (1996), 17,121-155).

At birth, but also throughout life until menopause, the majority of the ovarian follicles are in a dormant state. From its constitution, the oocyte capital progressively diminishes: thus, there are approximately 200 000 follicles per ovary at puberty, approximately 80 000 at 20 years of age, approximately 30 000 at 30 years of age, approximately 10 000 at 40 years of age, the capital being practically depleted at around 50 years of age (cf. Gougeon, A. and Lefevre, B., Médecine de la reproduction, 3rd edition, Ed Flammarion, p49).

The depletion of the oocyte capital corresponds clinically to menopause. The dormant follicles present in the ovary at a given time constitute the "ovarian reserve". Two mechanisms are involved in the progressive depletion of the ovarian reserve. Approximately 80% of the follicles disappear at the start via an apoptosis process, while the remaining 20% start to grow. The latter then begin a long process of development (approximately 6 months) in which a minority of them (approximately 400 over a lifetime) will arrive at the stage of preovulatory follicles containing a mature oocyte which is able to be fertilized (Gougeon A., Endocrine Reviews (1996), 17, 121-155). The majority of the growing follicles disappear through apoptosis leading to their involution; apoptosis strikes them at any stage of their development.

The change from the quiescent follicle stage to the growing follicle stage is a phenomenon which is continuous but of variable intensity. In particular, it accelerates in the 10 to 15 years preceding menopause, from approximately 38 years of age (Gougeon A., Endocrine Reviews (1996), 17, 121-155).

The factors stimulating the first stages of growth (starting from the large primary follicle) are relatively well known. They include gonadotropins luteinizing hormones (LH) and follicle-stimulating hormones (FSH) but particularly growth factors and steroids such as androgens. However, the mechanisms controlling the initiation of follicle growth are not well known. It is well established that this stage of folliculogenesis is not dependent on gonadotropins (LH and FSH) (cf. for example Bullun, S. and Adashi, E., Williams Textbook of Endocrinology, Tenth Edition (2003), 587-664).

In mammals, the mechanisms regulating initiation of follicle growth, a critical step for female reproduction, remain poorly understood. Whether follicle growth results from activation of resting follicles, from removal of an inhibition, or both, remains to be established.

Kit ligand (KL) is the first peptide that was demonstrated to trigger initiation of follicular growth. This is based on the analysis of the ovarian phenotype of animals bearing spontaneous inactivating mutations (Huang E.J. et al, Dev Biol 1993; 157:100-109) and experimental studies (Yoshida H. et al, Dev. Biol. 1997; 184:122-137, Parrott J.A. et al, Endocrinology 199; 140:4262-4271).

Basic fibroblast growth factor (bFGF) was found to induce primordial follicle development similar to what has been demonstrated for kit ligand/stem cell factor (KL). Basic FGF was localized to primordial and early developing follicles by immunocytochemistry and was primarily observed in the oocytes. Treatment of bovine ovarian theca cells and stroma cells with bFGF was found to promote cell growth (Skinner MK et al, Mol. Cell Endocrinol., 2001; 175; 123-130).

Somatostatin (SST) is a cyclic peptide present in two forms in the organism, one form containing 14 amino acids and one form containing 28 amino acids. The biological activity of these two forms of SST is similar. The SST-14 form is the predominant form in the central nervous system. It inhibits the secretion of the growth hormone by the somatotrope cells of the anterior pituitary. The SST-28 form is preferably expressed in the stomach and the pancreas. The biological activity of SST is induced by means of a series of membrane receptors coupled with a protein G, 5 sub-types of which have been characterized, namely the sub-types SSTR1 to SSTR5 (Reubi, J. C., Cancer Res., (1987) 47, 551-558; Resine, T.et al, Endocrin Reviews (1995) 16, 427-442; Lamberts, SW. et al., Endocrin Reviews (1991) 12, 450-482).

The presence of SST in the ovary has been demonstrated in several species including pigs (Mori, T. et al., Acta Endocrinol. (Copenh.) (1984), 106(2), 254-259), rats (McNeill, D. L. et al., Am. J Anat. (1987), 179(3), 269-76) and in women (Holst et al., Hum. Reprod. (1994), 9(8), 1448-1451). SST receptors have been identified in the ovary of the rat (Lidor, A. et al., Gynecol. Endocrinol. (1998), 12(2), 97-101) as well as in the human ovary in particular the sub-types 1, 2A and 5 (Strauss et al., Hum. Reprod. (2003), 18, Suppl. 1, P-495).

Some observations suggest that SST might play a role in maintaining follicles at the resting stage. SST is a peptide having a well-known effect in endocrine tissues: it inhibits cAMP generation (Ben-Shlomo et al., 2007) and it also inhibits KL production by sertoli cells (similar to granulosa cells) in the boar testis (Godard et al., 2001).

In the rat, SST, when administered *in vivo*, decreases the number of ovarian preovulatory follicles (Nestorovic et al., 2004), and, *in vitro*, SST inhibits both FSH-stimulated aromatase and progesterone production by granulosa cells (Andreani et al., 1995). In porcine granulosa cells, SST inhibits LH - and forskolin-stimulated production of cAMP (Rajkumar et al., 1992), and might inhibit the preovulatory oocyte nuclear maturation (Mori et al., 1985). In humans, SST directly inhibits both IGF -BP1 and progesterone synthesis by preovulatory granulosa cells (Holst et al., 1997). Following an *in vivo* treatment of patients with SST, LH release, androgen production and circulating levels of IGF-1 were reduced, whereas those of IGF-BP3 were increased (Fulghesu et al., 1995; Piaditis et al., 1996). When co-administered with FSH in patients suffering from a polycystic ovary syndrome, SST decreased circulating levels of LH, growth hormone and IGF-I, but did not affect FSH-stimulated follicular growth (Lidor et al., 1998; van der Meer et al., 1998). Taken together, these facts show that SST may have an effect on LH release and ovarian steroidogenesis in the mammalian ovary.

The above facts shows that SST is one of the factor, if not the sole, maintaining follicles at the resting stage. By inhibiting cAMP production by granulosa cells, SST may inhibit production of KL. Consequently, antagonists of the SSTR can stimulate entry of resting follicles into the growth phase.

The somatostatin receptor modulator analogs can be a cyclic or non-cyclic polypeptide, a fusion or recombination protein, a non-peptidic chemical entity (i.e. peptidomimetic, small molecules)) or also a "SS-like" peptide such as corticostatin.

In this manner, somatostatin modulator analogs initiate an increase in the number of follicles in the growth phase, which are therefore able to be stimulated with the standard treatments in order to reach the stage of pre-ovulatory follicles in women in need thereof.

Somatostatin modulator analogs can be used *in vitro* to induce early follicle growth in follicle cultures for the production of mature oocytes intended for fertilization. The somatostatin modulator analog is added to the culture media used to support *in vitro* follicle development. Moreover, the ability of the somatostatin modulator analogs to induce early follicle growth can also be used in the field of toxicological analyses. In particular, during tests of the effect of new chemical entities on follicle growth, the addition to the follicle sample of a somatostatin modulator analog makes it possible to accelerate follicle growth and thus to more easily observe any effect of slowing of said growth caused by said novel chemical entities.

There exists a set of clinical situations for which it is desirable from a medical perspective for the patient to slow the use of the ovarian reserve in order to delay the depletion of the latter and therefore to preserve the ovarian function and fertility. These situations are typically, and in a non-exclusive manner, patients at risk of early menopause that can be predicted on the basis of family antecedents, or genetic anomalies such as Turner syndrome (complete or partial). In this situation, the administration of a somatostatin modulator analog is a preventive measure and aims to slow the start of growth of the quiescent follicles.

The same applies for patients having difficulty conceiving and for whom the chronological age or biological age of their ovaries corresponds to the period of acceleration of activation of the quiescent follicles.

Another clinical situation which may benefit from a treatment by somatostatin modulator analog is the graft (preferably an autograft) of an ovary or of ovary fragments. In this context, the resumption of ovarian function is often temporary and is accompanied by a premature depletion of the number of primordial follicles (Baird, D. T. et al., Endocrinology (1999), 140, 462-471). It has in fact been demonstrated that, during the transplantation, the granulosa cells of the growing follicles are more inclined to start apoptosis than those of the primordial follicles (Liu, J. et al., Hum. Reprod. (2002), 17, 605-611). Moreover, the removal of ovarian tissue and its fragmentation causes the primordial follicles to move rapidly and en masse toward a stage of late primary follicles (cf. Wandji S-A, et al., Hum. Reprod. (1997), 12, 1993-2001; cf. also the control group of the example of the present application).

Somatostatin modulator analogs can be added to the various media for sampling, washing, preserving, freezing and thawing of ovarian tissue for the purpose of grafts. They can be used as a protective adjuvant for the media for sampling, washing, preserving, freezing and thawing of ovarian tissue.

The use of somatostatin modulator analog in patients with polycystic ovaries is also beneficial. In fact, numerous observations suggest that polycystic ovaries have an abnormally high follicle population (cf. Hughesdon, Obstet. Gynecol. Surv. (1982), 37(2), 59-77). The excessive production of androgens by these follicles in an excessive quantity could be the origin of the metabolic and endocrine disorders observed in these patients. Moreover, a significant reduction of this follicle population by ovarian resection, cauterization or ultraviolet radiation, constitutes one of the most effective therapies because it allows the patients to ovulate at approximately 80% and to become pregnant at a cumulative rate of 75% over 18 months. Thus, the regular administration of a somatostatin modulator analog should bring about a reduction in the number of growing follicles and therefore of the supernumerary antral follicles producing androgens resulting in the resumption of fertile ovulatory cycles of a temporary or permanent nature.

Moreover, the use of somatostatin modulator analog in patients who are about to have, are currently having or have had chemotherapy or irradiation (for therapeutic or other purposes) reduces the risk of early menopause by preventing the accelerated activation of the follicular reserve which would make it more sensitive to the chemotherapy agents or to the ionizing radiation.

Other applications can also be envisaged, in particular in the veterinary field. The invention could be used to save species, the use of somatostatin modulator analogs allowing the ovarian reserve of females to be preserved. Similarly, somatostatin modulator analogs can be used in the context of *in vitro* or *in vivo* fertilizations in animals of high commercial value. Such animals with high commercial value can in particular be horses, bovines, ovines or goats; they can also be animals of transgenic origin.

The patent application WO2005034989 discloses the use of somatostatin analogs, in the regulation of the ovarian follicular reserve. The invention primarily relates to the use of somatostatin or one of the agonistic analogs to regulate the ovarian follicular reserve or to the use of an antagonistic analog of somatostatin to accelerate the start of growing of quiescent follicles in non-menopausal women. Several classes of somatostatin antagonist analogs and agonist analogs are described but none of them have been claimed to have a dual agonist/antagonist profile.

It is reported that in neonatal mouse ovary a somatostatin receptor antagonist triggered a reduction of the percentages of primordial follicles and an increase of the percentages of primary and secondary follicles (A. Gougeon et al., 2010).

The classes of somatostatin receptor ligand antagonists or agonists disclosed in WO2005034989 do not encompass nor disclose Compound Cpa-[D-Cys-4-Pal-D-Trp-Lys-Thr-Cys]-2-Nal-NH₂ (Compound "A") according to the invention. A representative somatostatin antagonist analog described in WO2005034989 is an N-alkylated peptide of formula Cpa-[D-Cys-4-Pal-D-Trp-N-Me-Lys-Thr-Cys]-2-Nal-NH₂ (Compound "B"). The only difference between Compound "A" and the representative compound of WO2005034989 is the methyl-group attached to the nitrogen of the amide moiety of lysine.

Compound Cpa-[D-Cys-4-Pal-D-Trp-Lys-Thr-Cys]-2-Nal-NH₂ (Compound "A") was first described in WO2002070555. This patent application discloses the biological activity (i.e. somatostatin agonistic and antagonistic activity on somatostatin receptor subtypes) of several cyclic and non-cyclic peptides. The patent application further discloses methods for modulating the rate of proliferation of medullary thyroid carcinoma cells using cyclic peptides. Thus, the usage of Compound "A" disclosed in WO2002070555 is distinct from that of the present invention in so far as it is confined to the treatment of cancer.

The Applicants have found in a surprising manner that Compound "A" is relevant for accelerating the recruitment and the maturation of human ovarian follicle from the follicular reserve and for slowing down the depletion of the ovarian reserve, as well.

At low dose, Compound "A" pharmacological effect is dominated by its antagonistic property resulting into a reduction of the proportion of primordial follicles when compared to ovarian follicles from the control animals and into an increase of the proportion of primary, secondary and pre-antral and antral follicles, when compared to ovarian follicles from the control animals.

At high dose, Compound "A" pharmacological effect is dominated by its agonistic property resulting into a slowdown of the apoptosis of the resting follicles and a protection of the ovarian reserve.

In order to describe the *in vitro* actions of Compound "A" and to establish its effect on primordial follicle development, Applicants accomplished assays on rat ovarian culture systems. The results of both *in vitro* measurements were compared to that of Compound "B", a structurally similar peptide disclosed in the patent application WO2005034989.

It was established by the investigation of the number of primordial follicles and developing follicles that the actions of the two analogs are distinct from each other and Compound "A" shows a similar action to that of positive controls of KL and bFGF. This difference was demonstrated also on a molecular level gene expression.

The beneficial effects of Compound "A" were further investigated in a monkey *in vivo* model. The close similarity of the reproductive system, particularly with respect to ovarian morphology and ovarian cycle physiology and endocrinology, in cynomolgus macaques and in women makes the cynomolgus monkey a useful model for studies on ovarian cycle control, anovulatory fertility and ovarian reserve (Buse E., Zöller M. and Van Esch E. The Macaque Ovary, with Special Reference to the Cynomolgus Macaque (Macaca fascicularis). Toxicol Pathol 2008. 36: 24S).

According to the results of the above investigation, the Applicants have found, in a surprising manner, that the dual somatostatin receptor agonist/antagonist compound Cpa-[D-Cys-4-Pal-D-Trp-Lys-Thr-Cys]-2-Nal-NH₂ (Compound "A") has the capacity to modulate the somatostatin receptors' responses (Somatostatin Receptor Modulator), translating into a dual pharmacological effect on the ovarian reserve: 1) an acceleration of the start of growth of the quiescent follicles and/or 2) a protection of the quiescent follicles from apoptosis.

The administration of a medicament comprising Compound "A" and/or its non N-alkylated analogs in women with reproductive age leads to a slow down of the depletion of the ovarian reserve and/or an increase in the number of follicles in the growth phase and which are therefore able to be stimulated with the standard treatments in order to reach the stage of pre-ovulatory follicles.

The benefit of this discovery resides primarily in the possibility of using Compound "A" and its analogs in the treatment of infertility.

### SUMMARY OF THE INVENTION

The present invention provides a new use of a peptide derivative, compound Cpa-[D-Cys-4-Pal-D-Trp-Lys-Thr-Cys]-2-Nal-NH₂ (Compound "A") and its analogs, that have the ability to accelerate the growth of quiescent follicles from the follicular reserve and in the protection of the ovarian reserve from apoptosis, as well. Due to this dual pharmacological activity Compound "A" and its analogs are suitable for the treatment of infertility.

Accordingly, in one aspect the invention provides Compound Cpa-[D-Cys-4-Pal-D-Trp-Lys-Thr-Cys]-2-Nal-NH₂ (Compound "A") and/or its non N-alkylated analogs, any pharmaceutically acceptable salt thereof or an active metabolite thereof for use to accelerate the start of growth of quiescent follicles and/or for use to slow down the depletion of the ovarian reserve.

In a second aspect the invention provides a pharmaceutical composition comprising Compound Cpa-[D-Cys-4-Pal-D-Trp-Lys-Thr-Cys]-2-Nal-NH₂ (Compound "A") and/or its non N-alkylated analogs, any pharmaceutically acceptable salt thereof or an active metabolite thereof, for use to accelerate the start of growth of quiescent follicles and/or for use to slow down the depletion of the ovarian reserve.

In a third aspect the invention provides a pharmaceutical pack or kit comprising one or more containers filled with Compound Cpa-[D-Cys-4-Pal-D-Trp-Lys-Thr-Cys]-2-Nal-NH₂ (Compound "A") and/or its non N-alkylated analogs, any pharmaceutically acceptable salt thereof or an active metabolite thereof.

In a fourth aspect the invention provides a method for the acceleration the start of growth of quiescent follicles and/or for slowing down the depletion of the ovarian reserve comprising administering Compound Cpa-[D-Cys-4-Pal-D-Trp-Lys-Thr-Cys]-2-Nal-NH₂ (Compound "A") and/or its non N-alkylated analogs, any pharmaceutically acceptable salt thereof or an active metabolite thereof.

### DETAILED DESCRIPTION OF THE INVENTION

The present invention relates to Compound Cpa-[D-Cys-4-Pal-D-Trp-Lys-Thr-Cys]-2-Nal-NH₂ (Compound "A") and/or its non N-alkylated analogs, any pharmaceutically acceptable salt thereof or an active metabolite thereof for use to accelerate the start of growth of quiescent follicles and/or for use to slow down the depletion of the ovarian reserve.

In a preferred embodiment the invention relates to Compound Cpa-[D-Cys-4-Pal-D-Trp-Lys-Thr-Cys]-2-Nal-NH₂ (Compound "A") and/or its non N-alkylated analogs, any pharmaceutically acceptable salt thereof or an active metabolite thereof, for use to accelerate the start of growth of quiescent follicles and/or for use to slow down the depletion of the ovarian reserve in order to treat infertility.

In a still preferred embodiment the invention relates to Compound Cpa-[D-Cys-4-Pal-D-Trp-Lys-Thr-Cys]-2-Nal-NH₂ (Compound "A") and/or its non N-alkylated analogs, any pharmaceutically acceptable salt thereof or an active metabolite thereof, for use to accelerate the start of growth of quiescent follicles and/or for use to slow down the depletion of the ovarian reserve, wherein said Compound "A", any pharmaceutically acceptable salt thereof or an active metabolite thereof is to be administered to a mammal, preferably to a human being.

In a more preferred embodiment the invention relates to Compound Cpa-[D-Cys-4-Pal-D-Trp-Lys-Thr-Cys]-2-Nal-NH₂ (Compound "A") and/or its non N-alkylated analogs, any pharmaceutically acceptable salt thereof or an active metabolite thereof, for use to accelerate the start of growth of quiescent follicles and/or for use to slow down the depletion of the ovarian reserve, wherein said human being is a woman of reproductive age, women undergoing Assisted Reproductive Treatment and preferably women undergoing Assisted Reproductive Treatment but are poor responders to FSH stimulation.

In a further embodiment the invention relates to Compound Cpa-[D-Cys-4-Pal-D-Trp-Lys-Thr-Cys]-2-Nal-NH₂ (Compound "A") and/or its non N-alkylated analogs, any pharmaceutically acceptable salt thereof or an active metabolite thereof, for use to accelerate the start of growth of quiescent follicles and/or for use to slow down the depletion of the ovarian reserve, wherein said Compound Cpa-[D-Cys-4-Pal-D-Trp-Lys-Thr-Cys]-2-Nal-NH₂ (Compound "A") and/or its non N-alkylated analogs, or any pharmaceutically acceptable salt thereof or a pharmaceutical formulation thereof is to be administered orally, or parenterally, preferably subcutaneously.

In an other embodiment the invention relates to Compound Cpa-[D-Cys-4-Pal-D-Trp-Lys-Thr-Cys]-2-Nal-NH₂ (Compound "A") and/or its non N-alkylated analogs, any pharmaceutically acceptable salt thereof or an active metabolite thereof, for use to accelerate the start of growth of quiescent follicles and/or for use to slow down the depletion of the ovarian reserve, wherein said Compound Cpa-[D-Cys-4-Pal-D-Trp-Lys-Thr-Cys]-2-Nal-NH₂ (Compound "A") and/or its non N-alkylated analog, or any pharmaceutically acceptable salt thereof or an active metabolite thereof is administered daily, weekly or monthly.

In an other preferred embodiment the invention relates to Compound Cpa-[D-Cys-4-Pal-D-Trp-Lys-Thr-Cys]-2-Nal-NH₂ (Compound "A") and/or its non N-alkylated analogs, any pharmaceutically acceptable salt thereof or an active metabolite thereof, for use to accelerate the start of growth of quiescent follicles and/or for use to slow down the depletion of the ovarian reserve, wherein said Compound "A" and/or its non N-alkylated analog or any pharmaceutically acceptable salt thereof or an active metabolite thereof is administered in a daily dosage of 1 to 500 mg, preferably a daily dosage of 10mg to 200mg.

In a more preferred embodiment the invention relates to Compound Cpa-[D-Cys-4-Pal-D-Trp-Lys-Thr-Cys]-2-Nal-NH₂ (Compound "A") and/or its non N-alkylated analogs, any pharmaceutically acceptable salt thereof or an active metabolite thereof, for use to accelerate the start of growth of quiescent follicles and/or for use to slow down the depletion of the ovarian reserve, wherein the acceleration of the start of growth of quiescent follicles is for *in vitro* use in follicle cultures for the production of mature oocytes intended for fertilization.

In a most preferred embodiment the invention relates to Compound Cpa-[D-Cys-4-Pal-D-Trp-Lys-Thr-Cys]-2-Nal-NH₂ (Compound "A") and/or its non N-alkylated analogs, any pharmaceutically acceptable salt thereof or an active metabolite thereof, for use to accelerate the start of growth of quiescent follicles and/or for use to slow down the depletion of the ovarian reserve, wherein the acceleration of the start of growth of quiescent follicles is for use in toxicological analyses.

The present invention also relates to a pharmaceutical composition comprising Compound Cpa-[D-Cys-4-Pal-D-Trp-Lys-Thr-Cys]-2-Nal-NH₂ (Compound "A") and/or its non N-alkylated analogs, any pharmaceutically acceptable salt thereof or an active metabolite thereof, for use to accelerate the start of growth of quiescent follicles and/or for use to slow down the depletion of the ovarian reserve.

In a preferred embodiment the invention relates to a pharmaceutical composition comprising Compound Cpa-[D-Cys-4-Pal-D-Trp-Lys-Thr-Cys]-2-Nal-NH₂ (Compound "A") and/or its non N-alkylated analogs, any pharmaceutically acceptable salt thereof or an active metabolite thereof for use to accelerate the start of growth of quiescent follicles and/or for use to slow down the depletion of the ovarian reserve, in order to the treatment of infertility.

In a still preferred embodiment the invention relates to a pharmaceutical composition comprising Compound Cpa-[D-Cys-4-Pal-D-Trp-Lys-Thr-Cys]-2-Nal-NH₂ (Compound "A") and/or its non N-alkylated analogs, any pharmaceutically acceptable salt thereof or an active metabolite thereof, for use to accelerate the start of growth of quiescent follicles and/or for use to slow down the depletion of the ovarian reserve, wherein said Compound Cpa-[D-Cys-4-Pal-D-Trp-Lys-Thr-Cys]-2-Nal-NH₂ (Compound "A") and/or its non N-alkylated analog, any pharmaceutically acceptable salt thereof or an active metabolite thereof is to be administered to a mammal, preferably to a human being.

In a more preferred embodiment the invention relates to a pharmaceutical composition comprising Compound "A", any pharmaceutically acceptable salt thereof or an active metabolite thereof, for use to accelerate the start of growth of quiescent follicles and/or for use to slow down the depletion of the ovarian reserve, wherein said human being is a woman of reproductive age, women undergoing Assisted Reproductive Treatment and preferably women undergoing Assisted Reproductive Treatment but are poor responders to FSH stimulation.

In a further embodiment the invention relates to a pharmaceutical composition comprising Compound Cpa-[D-Cys-4-Pal-D-Trp-Lys-Thr-Cys]-2-Nal-NH₂ (Compound "A") and/or its non N-alkylated analogs, any pharmaceutically acceptable salt thereof or an active metabolite thereof, for use to accelerate the start of growth of quiescent follicles and/or for use to slow down the depletion of the ovarian reserve, wherein said Compound Cpa-[D-Cys-4-Pal-D-Trp-Lys-Thr-Cys]-2-Nal-NH₂ (Compound "A") and/or its non N-alkylated analog, or any pharmaceutically acceptable salt thereof or a pharmaceutical formulation thereof is to be administered orally, or parenterally, preferably subcutaneously.

In an other embodiment the invention relates to a pharmaceutical composition comprising Compound Cpa-[D-Cys-4-Pal-D-Trp-Lys-Thr-Cys]-2-Nal-NH₂ (Compound "A") and/or its non N-alkylated analogs, any pharmaceutically acceptable salt thereof or an active metabolite thereof, for use to accelerate the start of growth of quiescent follicles and/or for use to slow down the depletion of the ovarian reserve, wherein said Compound Cpa-[D-Cys-4-Pal-D-Trp-Lys-Thr-Cys]-2-Nal-NH₂ (Compound "A") and/or its non N-alkylated analog or any pharmaceutically acceptable salt thereof or a pharmaceutical formulation thereof is administered daily, weekly or monthly.

In an other preferred embodiment the invention relates to a pharmaceutical composition comprising Compound Cpa-[D-Cys-4-Pal-D-Trp-Lys-Thr-Cys]-2-Nal-NH₂ (Compound "A") and/or its non N-alkylated analogs, any pharmaceutically acceptable salt thereof or an active metabolite thereof, for use to accelerate the start of growth of quiescent follicles and/or for use to slow down the depletion of the ovarian reserve, wherein said Compound Cpa-[D-Cys-4-Pal-D-Trp-Lys-Thr-Cys]-2-Nal-NH₂ (Compound "A") and/or its non N-alkylated analog or any pharmaceutically acceptable salt thereof or a pharmaceutical formulation thereof is administered in a daily dosage of 1 to 500 mg, preferably a daily dosage of 10mg to 200mg.

In a more preferred embodiment the invention relates to a pharmaceutical composition comprising Compound Cpa-[D-Cys-4-Pal-D-Trp-Lys-Thr-Cys]-2-Nal-NH₂ (Compound "A") and/or its non N-alkylated analogs, any pharmaceutically acceptable salt thereof or an active metabolite thereof, for use to accelerate the start of growth of quiescent follicles and/or for use to slow down the depletion of the ovarian reserve, wherein the acceleration of the start of growth of quiescent follicles is for *in vitro* use in follicle cultures for the production of mature oocytes intended for fertilization.

In a most preferred embodiment the invention relates to a pharmaceutical composition comprising Compound Cpa-[D-Cys-4-Pal-D-Trp-Lys-Thr-Cys]-2-Nal-NH₂ (Compound "A") and/or its non N-alkylated analogs, any pharmaceutically acceptable salt thereof or an active metabolite thereof, for use to accelerate the start of growth of quiescent follicles and/or for use to slow down the depletion of the ovarian reserve, wherein the acceleration of the start of growth of quiescent follicles is for use in toxicological analyses.

The present invention also relates to a pharmaceutical pack or kit comprising one or more containers filled with Compound Cpa-[D-Cys-4-Pal-D-Trp-Lys-Thr-Cys]-2-Nal-NH₂ (Compound "A") and/or its non N-alkylated analogs, any pharmaceutically acceptable salt thereof or an active metabolite thereof.

The present invention further relates to a method for the acceleration the start of growth of quiescent follicles and/or for slowing down the depletion of the ovarian reserve comprising administering Compound Cpa-[D-Cys-4-Pal-D-Trp-Lys-Thr-Cys]-2-Nal-NH₂ (Compound "A") and/or its non N-alkylated analogs, any pharmaceutically acceptable salt thereof or an active metabolite thereof.

In a preferred embodiment the invention relates to to a method for the acceleration the start of growth of quiescent follicles and/or for slowing down the depletion of the ovarian reserve comprising administering Compound Cpa-[D-Cys-4-Pal-D-Trp-Lys-Thr-Cys]-2-Nal-NH₂ (Compound "A") and/or its non N-alkylated analogs, any pharmaceutically acceptable salt thereof or an active metabolite thereof, for the treatment of infertility.

In a still preferred embodiment the invention relates to a method for the acceleration the start of growth of quiescent follicles and/or for slowing down the depletion of the ovarian reserve comprising administering Compound Cpa-[D-Cys-4-Pal-D-Trp-Lys-Thr-Cys]-2-Nal-NH₂ (Compound "A") and/or its non N-alkylated analogs, any pharmaceutically acceptable salt thereof or an active metabolite thereof, wherein said Compound Cpa-[D-Cys-4-Pal-D-Trp-Lys-Thr-Cys]-2-Nal-NH₂ (Compound "A") and/or its non N-alkylated analogs, any pharmaceutically acceptable salt thereof or an active metabolite thereof is to be administered to a mammal, preferably to a human being.

In a more preferred embodiment the invention relates to a method for the acceleration the start of growth of quiescent follicles and/or for slowing down the depletion of the ovarian reserve comprising administering Compound Cpa-[D-Cys-4-Pal-D-Trp-Lys-Thr-Cys]-2-Nal-NH₂ (Compound "A") and/or its non N-alkylated analogs, any pharmaceutically acceptable salt thereof or an active metabolite thereof, wherein said human being is woman of reproductive age, women undergoing Assisted Reproductive Treatment and preferably women undergoing Assisted Reproductive Treatment but are poor responders to FSH stimulation.

In a further embodiment the invention relates to a method for the acceleration the start of growth of quiescent follicles and/or for slowing down the depletion of the ovarian reserve comprising administering Compound Cpa-[D-Cys-4-Pal-D-Trp-Lys-Thr-Cys]-2-Nal-NH₂ (Compound "A") and/or its non N-alkylated analogs, any pharmaceutically acceptable salt thereof or an active metabolite thereof, wherein said Compound Cpa-[D-Cys-4-Pal-D-Trp-Lys-Thr-Cys]-2-Nal-NH₂ (Compound "A") and/or its non N-alkylated analogs, any pharmaceutically acceptable salt thereof or an active metabolite thereof is to be administered orally, or parenterally, preferably subcutaneously.

In an other embodiment the invention relates to a method for the acceleration the start of growth of quiescent follicles and/or for slowing down the depletion of the ovarian reserve comprising administering Compound Cpa-[D-Cys-4-Pal-D-Trp-Lys-Thr-Cys]-2-Nal-NH₂ (Compound "A") and/or its non N-alkylated analogs, any pharmaceutically acceptable salt thereof or an active metabolite thereof, wherein said Compound Cpa-[D-Cys-4-Pal-D-Trp-Lys-Thr-Cys]-2-Nal-NH₂ (Compound "A") and/or its non N-alkylated analogs, any pharmaceutically acceptable salt thereof or an active metabolite thereof is to be administered daily, weekly or monthly.

In an other preferred embodiment the invention relates to a method for the acceleration the start of growth of quiescent follicles and/or for slowing down the depletion of the ovarian reserve comprising administering Compound Cpa-[D-Cys-4-Pal-D-Trp-Lys-Thr-Cys]-2-Nal-NH₂ (Compound "A") and/or its non N-alkylated analogs, any pharmaceutically acceptable salt thereof or an active metabolite thereof, wherein said Compound Cpa-[D-Cys-4-Pal-D-Trp-Lys-Thr-Cys]-2-Nal-NH₂ (Compound "A") and/or its non N-alkylated analogs, any pharmaceutically acceptable salt thereof or an active metabolite thereof is to be administered in a daily dosage of 1 to 500 mg, preferably a daily dosage of 10mg to 200mg.

In a more preferred embodiment the invention relates to a method for the acceleration the start of growth of quiescent follicles and/or for slowing down the depletion of the ovarian reserve comprising administering Compound Cpa-[D-Cys-4-Pal-D-Trp-Lys-Thr-Cys]-2-Nal-NH₂ (Compound "A") and/or its non N-alkylated analogs, any pharmaceutically acceptable salt thereof or an active metabolite thereof, wherein the acceleration of the start of growth of quiescent follicles is for *in vitro* use in follicle cultures for the production of mature oocytes intended for fertilization.

In a most preferred embodiment the invention relates to a method for the acceleration the start of growth of quiescent follicles and/or for slowing down the depletion of the ovarian reserve comprising administering Compound Cpa-[D-Cys-4-Pal-D-Trp-Lys-Thr-Cys]-2-Nal-NH₂ (Compound "A") and/or its non N-alkylated analogs, any pharmaceutically acceptable salt thereof or an active metabolite thereof, wherein the acceleration of the start of growth of quiescent follicles is for use in toxicological analyses.

As used herein, by "somatostatin receptor modulator" is meant a compound that has a high binding affinity (e.g., Kᵢ of less than 100 nM, or preferably less than 10 nM, or more preferably less than 1 nM) for one or several somatostatin receptors; his binding affinity can readily been assessed by those skilled in the art.

Natural or native Somatostatin (SST) is a cyclic peptide present in two forms in the organism, one form containing 14 amino acids and one form containing 28 amino acids. The biological activity of these two forms of SST is similar. The SST-14 form is the predominant form in the central nervous system. It inhibits the secretion of the growth hormone by the somatotrope cells of the anterior pituitary. The SST-28 form is preferably expressed in the stomach and the pancreas. The biological activity of SST is induced by means of a series of membrane receptors coupled with a protein G, 5 sub-types of which have been characterized, namely the sub-types SSTR1, SSTR2, SSTR3, SSTR4 and SSTR5.

As used herein, the term "modulating" (or "modulate") refers to altering (negatively or positively) the early follicle growth and/or ovarian follicle atresia by administering a somatostatin receptor agonist/antagonist, a pharmaceutically acceptable salt thereof or an active metabolite thereof.

By "pharmaceutically acceptable salt" of the somatostatin receptor modulator, Compound "A" is meant, in particular in the present application, addition salts with inorganic acids such as hydrochloride, hydrobromide, hydroiodide, sulphate, phosphate, diphosphate and nitrate or with organic acids such as acetate, maleate, fumarate, tartrate, succinate, citrate, lactate, methanesulphonate, p-toluenesulphonate, palmoate and stearate. Also included in the field of the present invention, when they can be used, the salts formed from bases such as sodium or potassium hydroxide. For other examples of pharmaceutically acceptable salts, reference can be made to "Salt selection for basic drugs", Int. J. Pharm. (1986), 33, 201-217.

An "active metabolite" is a product produced through metabolism in the body of a specified compound or salt thereof and which exhibits the same biological activity as the specified compound. Active metabolites of a somatostatin receptor analog or of a salt of said somatostatin receptor analog may be identified using routine techniques known in the art and their activities determined using tests such as known in the art. Such metabolites may result for example from the oxidation, reduction, hydrolysis, amidation, deamidation, esterification, deesterification, enzymatic cleavage, monohydroxylation, dihydroxylation, methylation, glucuronidation, O-glucuronidation, sulfation, amide-hydrolysis, activation and the like, of the administered somatostatin receptor analog or of a salt of said somatostatin receptor analog. Accordingly, the invention includes active metabolites of somatostatin receptor analog or of a salt of said somatostatin receptor analog, including compounds produced by a process comprising contacting a compound of this invention with a mammal for a period of time sufficient to yield a metabolic product thereof. Such metabolite may also be produced *in vitro* by oxidation, reduction, hydrolysis, amidation, deamidation, esterification, deesterification, or enzymatic cleavage of the corresponding somatostatin receptor analog or salt of said somatostatin receptor analog.

The general term "analog" is commonly used to describe compounds derived from the native structure and selected for their enhanced antagonist/agonist activity. By "somatostatin analog" is thus meant a compound derived from the native structure of somatostatin and selected for its enhanced antagonist and/or agonist activity. A somatostatin analog can be either i) synthetic, i.e. obtained by chemical synthesis (e.g peptide synthesis) or ii) recombinant i.e. obtained by recombinant techniques, where the somatostatin analog amino acid sequence is encoded by a cloned gene and expressed and recovered from expression cells iii) peptidic or iv) non-peptidic.

### Compound "A": somatostatin receptor modulator of the chemical formula: Cpa-[D-Cys-4-Pal-D-Trp-Lys-Thr-Cys]-2-Nal-NH2

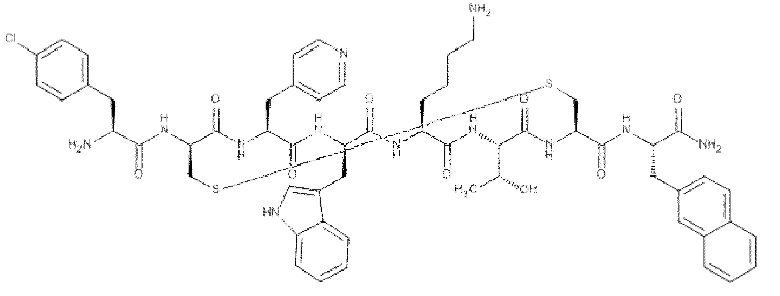

The somatostatin receptor modulator Compound "A" according to the invention is selected from the group comprising, but not limited to recombinant Compound "A" modulator analogs of somatostatin or synthetic Compound "A" modulator analogs of somatostatin.

A "non N-alkylated analog" of Compound "A" is a cyclic or non cyclic peptide derived from the structure of Compound "A" with each nitrogen atom of the peptide amide bond being not substituted with an alkyl group.

The term "pre-ovulatory follicles" is used to described growing follicles that are responsive to FSH stimulation.

The term "ovarian reserve" or "ovarian follicular reserve" is used to described the number of remaining primordial follicles in the ovaries.

The term "quiescent follicles" is used to describe dormant follicles or primordial follicles from the ovarian reserve.

"Ovarian follicle atresia" or "apoptosis" refers to a process through which ovarian follicles undergo a degenerative process called atresia during reproductive life. Almost 99% of ovarian follicles undergo this process and only 1% proceeds into ovulation the few that are selected to become ovulatory follicles are transformed into corpora lutea following ovulation.

The present invention also provides Compound "A", a pharmaceutically acceptable salt thereof or an active metabolite thereof, for its use to accelerate the maturation of the resting ovarian follicles from the follicular reserve and/or to preserve the ovarian reserve from rapid depletion, and therefore in infertility treatment.

As used herein, "patient in need thereof" is well-recognized in the art, and, is used herein to refer to a mammal and, more preferably, a human patient, and even more preferably a human female.

"A gynecological disease or condition" refers to a disease or condition related to the health of the female reproductive system (uterus, vagina, and ovaries).

As used herein, "treatment of infertility" relates to the treatment of conditions selected from the group comprising Incipiens ovarian failure, Premature ovarian failure, infertility, anovulation, infertility characterized by "poor ovarian response" to gonadotropin therapy, delayed puberty, infertility associated with elevated FSH levels, pre-treatment to IVF and ART (Assisted Reproductive Technology), spontaneous premature ovarian failure (early menopause), polycystic ovarian disease (reduction of the number of growing follicles), and bad responders to COS (controlled ovarian stimulation). As use herein, "treatment of infertility" relates also to the treatment using *in vitro* induction of early follicle growth in follicle cultures for the production of mature oocytes intended for fertilization.

Also contemplated is the use of a Compound "A", a pharmaceutically acceptable salt thereof or an active metabolite thereof, for preparing a medicament in the treatment and/or prevention of a gynaecological disease or condition selected from the group comprising Incipiens ovarian failure, Premature ovarian failure, infertility, anovulation, infertility characterized by "poor ovarian response" to gonadotropin therapy, delayed puberty, infertility associated with elevated FSH levels, pre-treatment to IVF and ART (Assisted Reproductive Technology), spontaneous premature ovarian failure (early menopause), polycystic ovarian disease (reduction of the number of growing follicles), and bad responders to COS (controlled ovarian stimulation).

### Administration:

According to the present invention, the pharmaceutical composition, preparation or medicament containing Compound "A", a pharmaceutically acceptable salt thereof or an active metabolite thereof, as applicable in this invention, can be administered by parenteral route (subcutaneous, intravenous, intramuscular, intraperitoneal or in an implant), by oral, vaginal, intrauterine, rectal, nasal, sublingual or transdermal route.

Preferred route of administration is subcutaneous.

One variation of the present invention also foresees a pharmaceutical composition or preparation suitable for oral administration of Compound "A", a pharmaceutically acceptable salt thereof or an active metabolite thereof as defined in the present invention. Compound "A", a pharmaceutically acceptable salt thereof or an active metabolite thereof, for example, may be orally administered in combination with a transient permeability enhancer allowing increase intestinal absorption. All transient permeability enhancement technology, well known by those skilled in the art are potential candidate to be used in this invention.

Solid dosage forms for oral administration include capsules, tablets, pills, powders and granules. In such solid dosage forms, the active compound may be mixed with at least one inert, pharmaceutically acceptable excipient or carrier, such as sodium citrate or dicalcium phosphate and/or (a) fillers or extenders such as starches, lactose, sucrose, glucose, mannitol and silicic acid; (b) binders such as carboxymethylcellulose, alginates, gelatin, polyvinylpyrrolidone, sucrose and acacia; (c) humectants such as glycerol; (d) disintegrating agents such as agar-agar, calcium carbonate, potato or tapioca starch, alginic acid, certain silicates and sodium carbonate; (e) solution retarding agents such as paraffin; (f) absorption accelerators such as quaternary ammonium compounds; (g) wetting agents such as cetyl alcohol and glycerol monostearate; (h) absorbents such as kaolin and bentonite clay; and (i) lubricants such as talc, calcium stearate, magnesium stearate, solid polyethylene glycols, sodium lauryl sulfate and mixtures thereof. In the case of capsules, tablets and pills, the dosage form may also comprise buffering agents.

Solid compositions of a similar type may also be employed as fillers in soft and hard-filled gelatin capsules using such excipients as lactose or milk sugar as well as high molecular weight polyethylene glycols and the like.

The solid dosage forms of tablets, capsules, pills and granules can be prepared with coatings and shells such as enteric coating and other coatings well-known in the pharmaceutical formulating art. They may optionally contain opacifying agents and may also be of a composition such that they release the active ingredient(s) only, or preferentially, in a certain part of the intestinal tract, optionally, in a delayed manner. Examples of embedding compositions which can be used include polymeric substances and waxes.

Liquid dosage forms for oral administration include pharmaceutically acceptable emulsions, solutions, suspensions, syrups and elixirs. In addition to the active compounds, the liquid dosage forms may contain inert diluents commonly used in the art such as, for example, water or other solvents, solubilizing agents and emulsifiers such as ethyl alcohol, isopropyl alcohol, ethyl carbonate, ethyl acetate, benzyl alcohol, benzyl benzoate, propylene glycol, 1,3-butylene glycol, dimethyl formamide, oils (in particular, cottonseed, groundnut, corn, germ, olive, castor and sesame oils), glycerol, tetrahydrofurfuryl alcohol, polyethylene glycols and fatty acid esters of sorbitan and mixtures thereof.

In cases where Compound "A", a pharmaceutically acceptable salt thereof or an active metabolite thereof is included in a solution, the formulation may contain suspending agents, as for example, ethoxylated isostearyl alcohols, polyoxyethylene sorbitol and sorbitan esters, microcrystalline cellulose, aluminum metahydroxide, bentonite, agar-agar and tragacanth, or mixtures of these substances, among others.

Useful intranasal formulations of Compound "A", a pharmaceutically acceptable salt thereof or an active metabolite thereof may contain stabilizers and surfactants. Among the pharmaceutically acceptable surfactants are polyoxyethylene castor oil derivatives, such as polyoxyethylene-glycerol-triricinoleate, also known as polyoxyl 35 castor oil (CREMOPHOR EL), or polyoxyl 40 hydrogenated castor oil (CREMOPHOR RH40) (both available from BASF Corp.); mono-fatty acid esters of polyoxyethylene (20) sorbitan, such as polyoxyethylene (20) sorbitan monolaurate (TWEEN 80), polyoxyethylene monostearate (TWEEN 60), polyoxyethylene (20) sorbitan monopalmitate (TWEEN 40), or polyoxyethylene (20) sorbitan monolaurate (TWEEN 20) (all available from ICI Surfactants of Wilmington, Del.); polyglyceryl esters, such as polyglyceryl oleate; and polyoxyethylated kernel oil (LABRAFIL, available from Gattefosse Corp.). Preferably, the surfactant will be between about 0.01% and 10% by weight of the pharmaceutical composition. Among the pharmaceutically useful stabilizers are antioxidants such as sodium sulfite, sodium metabisulfite, sodium thiosulfate, sodium formaldehyde sulfoxylate, sulfur dioxide, ascorbic acid, isoascorbic acid, thioglycerol, thioglycolic acid, cysteine hydrochloride, acetyl cysteine, ascorbyl palmitate, hydroquinone, propyl gallate, nordihydroguaiaretic acid, butylated hydroxytoluene, butylated hydroxyanisole, alpha-tocopherol and lecithin. Preferably, the stabilizer will be between about 0.01% and 5% by weight of the pharmaceutical composition.

Suspensions may also include chelating agents such as ethylene diamine tetraacetic acid, its derivatives and salts thereof, dihydroxyethyl glycine, citric acid and tartaric acid among others. Additionally, proper fluidity of a suspension can be maintained, for example, by the use of coating materials such as lecithin, by the maintenance of the required particle size in the case of dispersions and by the use of surfactants, such as those previously mentioned.

One embodiment of the present invention also foresees a pharmaceutical composition or preparation suitable for delayed and controlled release of Compound "A", a pharmaceutically acceptable salt thereof or an active metabolite thereof as defined in the present invention. The Compound "A", a pharmaceutically acceptable salt thereof or an active metabolite thereof, for example, may be incorporated in a matrix of biocompatible polymer allowing delayed and controlled release. All biocompatible polymers, well known by those skilled in the art are potential candidate to be used in this invention. Vaginal rings or intra-uterine devices are also contemplated options.

Sustained-release preparations may be prepared. Suitable examples of sustained-release preparations include semi permeable matrices of solid hydrophobic polymers containing Compound "A", a pharmaceutically acceptable salt thereof or an active metabolite thereof, which matrices are in the form of shaped articles, e.g. films, or microcapsules. Examples of sustained-release matrices include polyesters, hydrogels (for example, poly(2-hydroxyethyl-methacrylate), or poly(vinylalcohol)), polylactides (U.S. Pat. No. 3,773,919), copolymers of L-glutamic acid and [gamma] ethyl-L-glutamate, non-degradable ethylenevinyl acetate, degradable lactic acid-glycolic acid copolymers such as the LUPRON DEPOT(TM) (injectable microspheres composed of lactic acid-glycolic acid copolymer and leuprolide acetate), and poly-D-(-)-3-hydroxybutyric acid.

The formulations to be used for *in vivo* administration must be sterile. This is readily accomplished for example by filtration through sterile filtration membranes.

### Dose:

The dose of Compound "A" according to the present invention, intended for the use in the acceleration of the start of growth of quiescent follicles and a protection of the ovarian reserve from apoptosis in women in reproductive age and in the treatment of infertility, varies according to the method of administration, the age and body weight of the subject to be treated as well as the condition of the subject, and the final decision is made by the attending doctor or vet. Such a quantity determined by the attending doctor is called "therapeutically effective amount" here.

Usually, Compound "A", a pharmaceutically acceptable salt thereof or an active metabolite thereof will be administered, preferably daily. However, the appropriate period will depend notably on the age and body weight of the subject to be treated as well and the mode of administration and the formulation of Compound "A".

Alternatively, Compound "A", a pharmaceutically acceptable salt thereof or an active metabolite thereof will be administered, every other day, once a week or once every month, preferably, as a onetime administration.

Usually, Compound "A", a pharmaceutically acceptable salt thereof or an active metabolite thereof is administered at a daily dose between 0.1 to 1000 mg. However, the appropriate dosage will depend notably on the age and body weight of the subject to be treated as well and the mode of administration. Possible mode of administration include tablets, capsules, lozenges, pills, dental pastes, suppositories, inhalants, solutions, ointments, parenteral depots, vaginal rings and intra-uterine delivery systems.

Alternatively, or additionally, it will become apparent that the pharmaceutical composition or formulation described herein may be administered alone or in combination with other treatments, therapeutics or agents, either simultaneously or sequentially dependent upon the condition or disease to be treated. For example, Compound "A", a pharmaceutically acceptable salt thereof or an active metabolite thereof in the method of the invention may be administered in association with, e.g. a recombinant follitropin either simultaneously or sequentially.

### Carriers:

Compound "A", a pharmaceutically acceptable salt thereof or an active metabolite thereof for use in the method as described herein, are usually in the form of a pharmaceutical composition that may contain one or more pharmaceutically acceptable carriers, such as excipients, carriers and/or auxiliaries or stabilizers which facilitate processing of the active compounds into preparation which can be used pharmaceutically.

Acceptable carriers, excipients, or stabilizers are non-toxic to recipients at the dosages and concentrations employed, and include buffers such as phosphate, citrate, and other organic acids; antioxidants including ascorbic acid and methionine; preservatives (such as octadecyldimethylbenzyl ammonium chloride; hexamethonium chloride; benzalkonium chloride, benzethonium chloride; phenol, butyl or benzyl alcohol; alkyl parabens such as methyl or propyl paraben; catechol; resorcinol; cyclohexanol; 3-pentanol; and m-cresol); low molecular weight (less than about 10 residues) polypeptides; proteins, such as serum albumin, gelatin, or immunoglobulins; hydrophilic polymers such as polyvinylpyrrolidone; amino acids such as glycine, glutamine, asparagine, histidine, arginine, or lysine; monosaccharides, disaccharides, and other carbohydrates including glucose, mannose, or dextrins; chelating agents such as EDTA; sugars such as sucrose, mannitol, trehalose or sorbitol; salt-forming counter-ions such as sodium; metal complexes (e.g. Zn-protein complexes); and/or non-ionic surfactants such as TWEEN®, PLURONICS® or polyethylene glycol (PEG).

### Method of treating:

The present invention also provides for a method of treating and/or preventing infertility in a patient in need thereof comprising administering to said patient a pharmaceutical composition comprising Compound "A", a pharmaceutically acceptable salt thereof or an active metabolite thereof.

### Kit:

The present invention also contemplates a kit for treating and/or preventing a gynecological disease or condition comprising Compound "A", any pharmaceutically acceptable salt thereof or or an active metabolite thereof, or a pharmaceutical formulation thereof optionally with reagents and/or instructions for use.

Generally, the Kit comprises a container and a label or package insert on or associated with the container. Suitable containers include, for example, bottles, vials, syringes, etc. The containers may be formed from a variety of materials such as glass or plastic. The container holds Compound "A", a pharmaceutically acceptable salt thereof or an active metabolite thereof of the invention which is effective for treating and/or preventing a gynecological disease or condition and may have a sterile access port (for example the container may be an intravenous solution bag or a vial having a stopper pierceable by a hypodermic injection needle or an aerosol spray device). The label or package insert indicates that the composition is used for treating the condition of the invention.

Alternatively, or additionally, the Kit may further comprise a second (or third) container comprising a pharmaceutically acceptable buffer, such as bacteriostatic water for injection (BWFI)5 phosphate-buffered saline, Ringer's solution and dextrose solution. It may further include other materials desirable from a commercial and user standpoint, including other buffers, diluents, filters, needles, and syringes.

Those skilled in the art will appreciate that the invention described herein is susceptible to variations and modifications other than those specifically described. It is to be understood that the invention includes all such variations and modifications without departing from the spirit or essential characteristics thereof. The invention also includes all of the steps, features, compositions and compounds referred to or indicated in this specification, individually or collectively, and any and all combinations or any two or more of said steps or features. The present disclosure is therefore to be considered as in all aspects illustrated and not restrictive, the scope of the invention being indicated by the appended Claims, and all changes which come within the meaning and range of equivalency are intended to be embraced therein.

Various references are cited throughout this Specification, each of which is incorporated herein by reference in its entirety.

The foregoing description will be more fully understood with reference to the following Examples. Such Examples are, however, exemplary of methods of practicing the present invention and are not intended to limit the scope of the invention.

### DESCRIPTION OF THE FIGURES

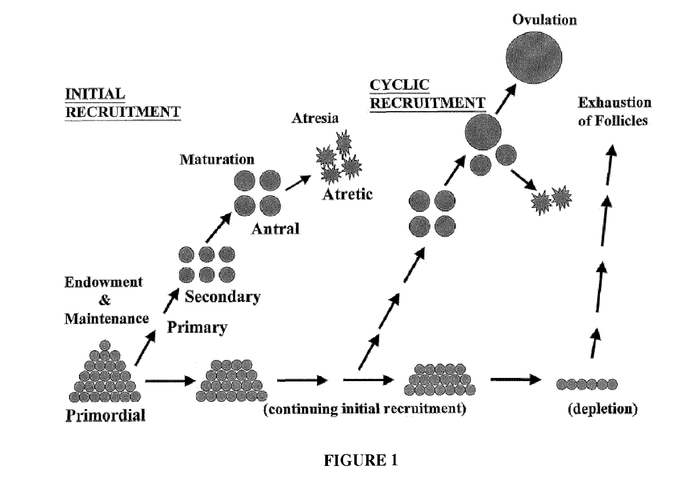

**FIGURE 1** **: Life history of ovarian follicles**
   A fixed number of primordial follicles are endowed during early life, and most of them are maintained in a resting state. Growth of some of these dormant follicles is initiated before and throughout reproductive life (Initial recruitment). Follicles develop through primordial, primary, and secondary stages before acquiring an antral cavity. At the antral stage most follicles undergo atresia; however, under optimal gonadotropin stimulation that occurs after puberty, a few of them are rescued (Cyclic recruitment) to reach the preovulatory stage. Eventually, depletion of the pool of resting follicles leads to ovarian follicle exhaustion and senescence.

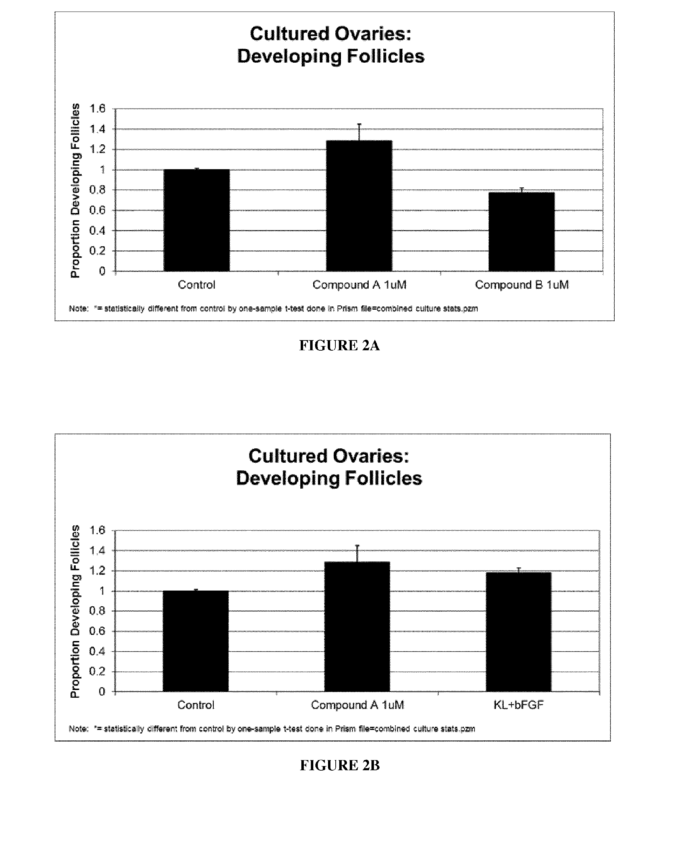

**FIGURE 2A & 2B** **: Histological analysis of 4-day post-natal rat ovaries cultured for 10 days in the presence or absence of somatostin receptor modulator compounds "A" and somatostatin receptor antagonist "B"**
   A positive control of Kit Ligand (KL) and basic fibroblast growth factor (bFGF) is used and shown to stimulate the primordial to primary follicle transition approximately 20% during the 10-day organ culture. Compound "A" also stimulated primordial follicle development while there is no significant effect of Compound "B" on the primordial follicle development at the same concentration.
**FIGURE 3A****: Dendogram for Compound "A" and Compound "B" affected genes**
**FIGURE 3B****: Venn diagram of the number of genes differentially expressed (p<0.05) between treated (Compound "A" or Compound "B") and control ovaries**
   The data from FIGURE 3A and 3B demonstrate that the actions of the two analogs "A" and "B" were distinct from each other on the gene level, supporting different biological actions for the two analogs.

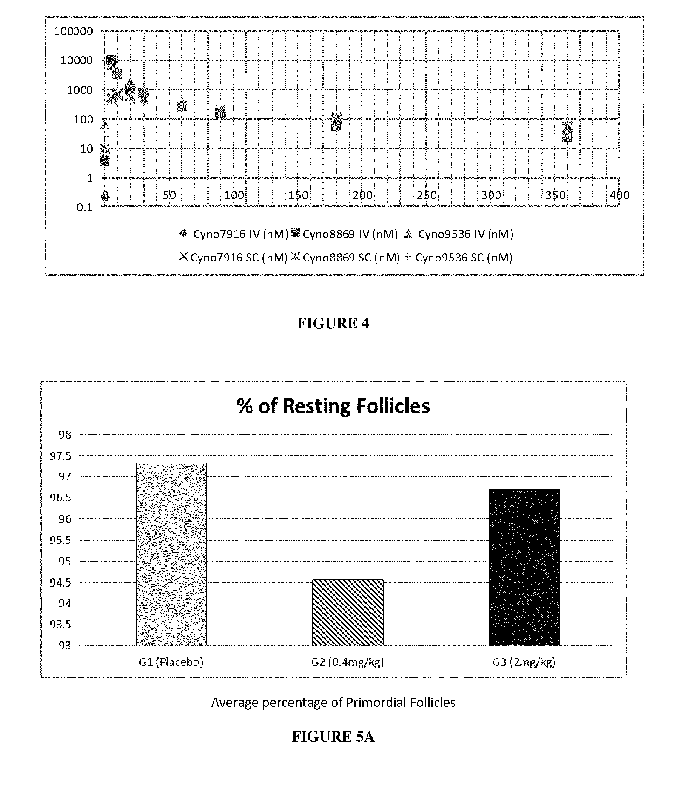

**FIGURE 4** **: Pharmacokinetics of Compound "A" in young adult female Cynomolgus monkeys via intravenous (IV) mode of administration or via subcutaneous (SC) mode of administration**
   When administered at 2mg/kg, Compound "A" has a similar plasma exposure intravenously than via the subcutaneous route.

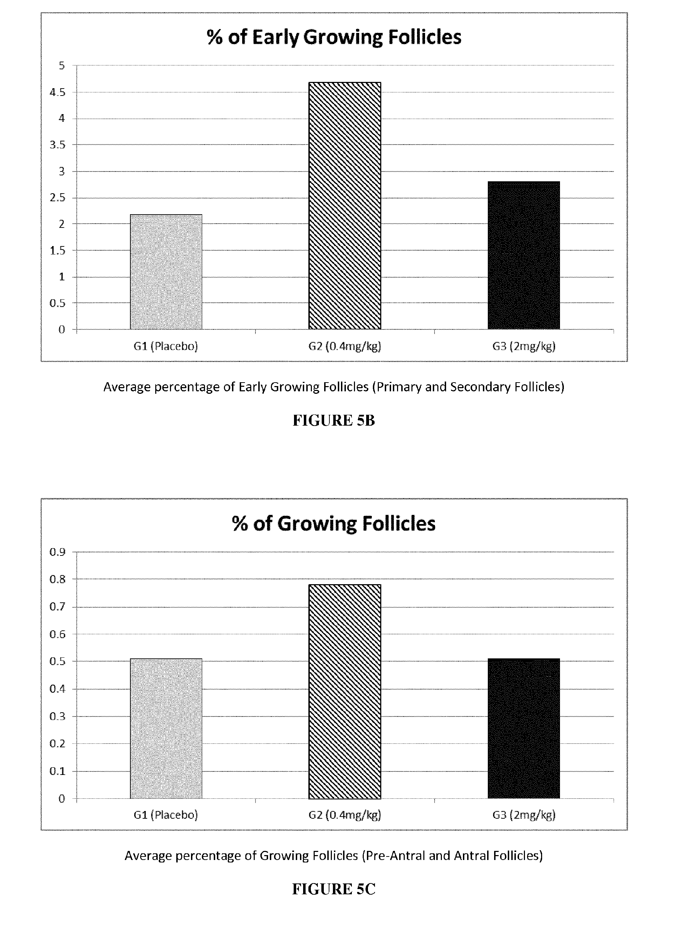

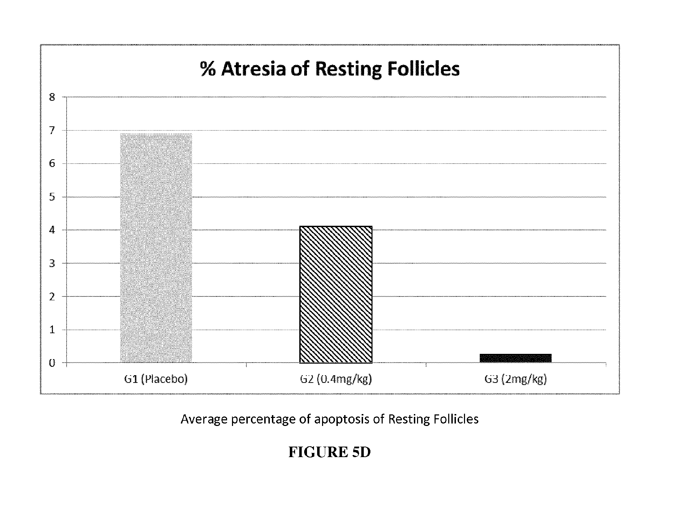

**FIGURE 5A****,** **5B****,** **5C** **&** **5D****: Histological quantification of changes in follicle development in ovaries from young adult female cynomolgus monkeys treated with the somatostatin modulator Compound "A"**
   At low dose, Compound "A" pharmacological effect is dominated by its antagonistic property resulting into a reduction of the proportion of primordial follicles when compared to ovarian follicles from the control animals and into an increase of the proportion of primary, secondary and pre-antral and antral follicles, when compared to ovarian follicles from the control animals.
   At high dose, Compound "A" pharmacological effect is dominated by its agonistic property resulting into a slowdown of the apoptosis of the resting follicles and a protection of the ovarian reserve.

### EXAMPLES

### EXAMPLE 1: Synthesis of Compound "A"

Compound "A" is synthesized by solid phase chemistry, using standard Fmoc-protection strategy and purified by reverse-phase HPLC, reaching purity desired. The general process is as follows:
**The sequence of Compound "A":** Cpa-DCys-4Pal-DTrp-Lys-Thr-Cys-2Nal-NH2 (DCys&Cys Bridge)
**The general process:**
   ① **Fmoc-2Nal-OH**(useRink Amide MBHA-resin to link)→
   ② Add **Fmoc-Cys(Trt)-OH**→
   ③ Then dehydrate and add **Fmoc-Thr(tBu)-OH**→
   ④ Then dehydrate and add **Fmoc-Lys(Boc)-OH**→
   ⑤ Then dehydrate and add **Fmoc-D-Trp(Boc)-OH**→
   ⑥ Then dehydrate and add **Fmoc-4Pal-OH**→
   ⑦ Then dehydrate and add **Fmoc-D-Cys(Trt)-OH**→
   ⑧ Then dehydrate and add **Fmoc-Cpa-OH**→
   ⑨ Then dehydrate and cleavage of Fmoc (Fluorenylmethyloxycarbonyl) and Boc (tert-butyl dicarbonate) protecting group from NH2-resin
   ⑩ Crude peptide oxide to get disulfide bridge between **DCys and Cys**→ Then purified by reverse-phase HPLC to reach the purity desired.

### EXAMPLE 2: Comparison of Cpa-[D-Cys-4-Pal-D-Trp-Lys-Thr-Cysl-2-Nal-NH₂ (Compound "A") and Cpa-[D-Cys-4-Pal-D-Trp-N-Me-Lys-Thr-Cys]-2-Nal-NH₂ (Compound "B")

### In vitro profile:

Based on *in vitro* binding assays and *in vitro* functional assay, Compound "A" is a strong antagonist of somatostatin receptor 2 and an agonist of somatostatin receptor 5 while Compound "B" is reported in WO2005034989 to be an antagonist of the somatostatin receptors.

| Peptide | hsst1 Ki(nM) | hsst2 Ki(nM) | hsst3 Ki(nM) | hsst4 Ki(nM) | hsst5 Ki(nM) | Functional assay (Calcium mobilisation) |
|---|---|---|---|---|---|---|
| ***Compound* "*A*"** | 2757 | **6.4** | 44 | 711 | **86** | **Strong antagonist of SSTR2 Agonist of SSTR5** |
| ***Compound* "*B*"** | 1000 | **17.1** | 66 | 1000 | **6** | **Antagonist** |

### Effect on primordial follicle development:

The *in vitro* actions of Compound "A" and Compound "B" on rat primordial follicle development were also investigated with an ovarian organ culture system. Two procedures were used to assess the actions of the analogs. The first was to culture for 10 days the postnatal day 4 rat ovaries in the absence or presence of the analogs followed by a histological analysis of the ovary. The number of primordial follicles and developing follicles was determined to assess actions of the analogs on the induction of primordial follicle development. The second procedure was to culture for only 2 days the postnatal day 4 rat ovaries in the absence or presence of the analogs, followed by RNA collection from the ovary sample and analysis of the ovarian transcriptome with a microarray analysis. This experimental design determines the effects of the somatostatin analogs "A" and "B" on primordial follicle development using a morphological analysis of the follicle, and also determines the ability of the analogs to alter gene expression after a short treatment, prior to any change in follicle development, to assess the early event responses of the ovary to the analogs.

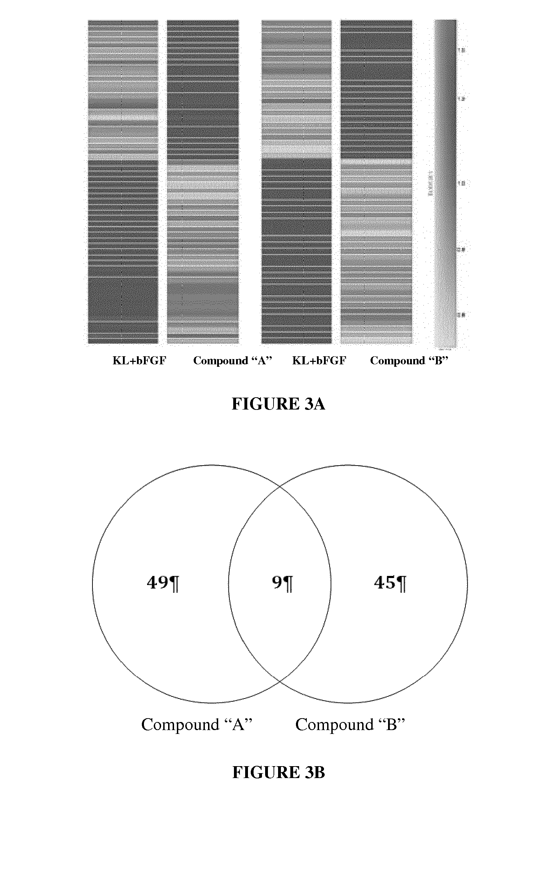

The somatostatin analogs "A" and "B" did effect primordial follicle development in a different manner in a rat ovary organ culture procedure. The magnitude of the stimulatory effect of Compound "A" was similar to the positive controls of KL and bFGF while there is no significant effect of Compound "B" on the primordial follicle development (see FIGURES 2A and 2B). Therefore, the results demonstrated that Compound "A" stimulate the transition of primordial follicles to primary follicles. The ability of the analogs "A" and "B" to alter on a molecular level gene expression of the ovary, also supports the conclusion that the ovary is directly effected by the analogs (see FIGURES 3A and 3B). Surprisingly, the actions of the two analogs "A" and "B" were distinct from each other, again supporting different actions for the two. From the above facts the conclusion can be drawn that the somatostatin analogs do alter on a molecular level ovary function, which appears to correlate to the morphological effects observed on primordial follicle development.

The data above demonstrated that non N-alkylated peptides somatostatin analogs such as Compound "A" could be used as somatostatin receptor modulators and have a more appropriate biological profile than N-alkylated peptides somatostatin analogs for the optimal regulation of the ovarian follicular reserve and thus treatment of infertility.

### EXAMPLE 3: Ovarian Reserve Assessment Following Somatostatin Modulator Compound "A" Administration in Young Adult Female Cynomolgus Macaques

### Study Design:

The main PD study was run for at least 97 days inclusive of a one-week pre-dosing period to obtain baseline observations and measurements. The first day of dosing was designated as Day 1. Dosing was carried out twice daily for 90 days.

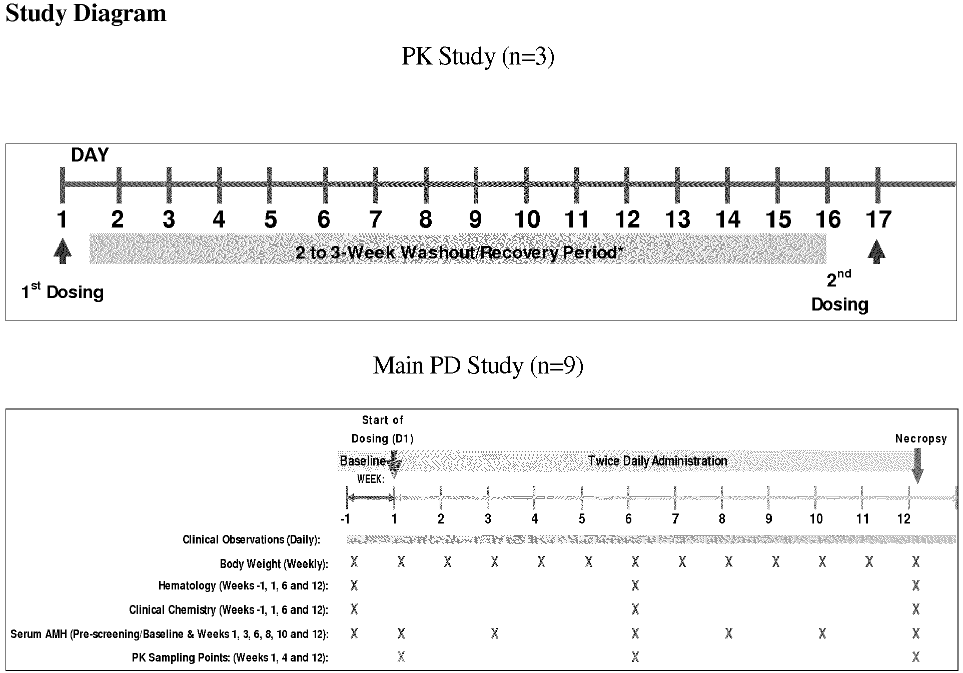

### Dose Design and Administration of Test and Control Substances

For the preliminary PK study phase, 3 female cynomolgus monkeys were selected and received the test substance, Compound "A" as outlined below. The test substance was administered intravenously at 2 mg/kg on the first day. Following a 2- to 3-week washout/recovery period, a second administration at 2 mg/kg via the subcutaneous (SC) route was performed.

**PK Dose Design**

| Schedule | Test Substance | Dose Level (mg/kg) | Dose Volume (mL/kg) | Concentration (mg/mL) | Route of Administration | Number of Animals |
|---|---|---|---|---|---|---|
| 1^{st} Dosing | Compound "A" | 2 | 0.67 | 3 | IV | 3 |
| 2- to 3-Week Washout/Recovery Period | | | | | | |
| 2^{nd} Dosing | Compound "A" | 2 | 0.67 | 3 | SC | |

For the main PD study, nine (9) young adult female cynomolgus monkeys, which included the 3 animals that have been included to the PK study, was assigned to one of 3 dose groups (Groups 1, 2 and 3) as shown in the table below. Stratification was done based on their baseline level of AMH.

The dose volume was adjusted to the latest body weight recorded prior to dosing. Immediately post injection of test substance, 0.5 mL of the vehicle was administered to flush the system.

**Main Pharmacodynamic Study Dose Design**

| Group | Test Substance | Dose Level (mg/kg) | Dose Volume (mL/kg) | Concentration (mg/mL) | Route of Administration | Number of Animals |
|---|---|---|---|---|---|---|
| 1 | Vehicle | 0 | - | 0 | SC | 3 |
| 2 | Compound "A" | 0.4* (Low) | 0.67 | 0.6 | SC | 3 |
| 3 | Compound "A" | 2 (High) | 0.67 | 3 | SC | 3 |

All animals received subcutaneous injections of Compound "A" or the vehicle twice daily for 90 days.

### Material and methods:

### Vehicle / Control

The vehicle/control substance, which is mixture of saline, 0.1 N HCl, heat-inactivated monkey serum, and dimethylsulfoxide (DMSO), was prepared. The heat inactivated monkey serum was either prepared or supplied from a commercial source.

### Heat inactivation of monkey serum

Cynomolgus serum was thawed and placed in a 56 °C water bath for 30 min with occasional stirring. The serum was allowed to cool slowly to room temperature and then centrifuged at 13000 rpm for 10 minutes at room temperature in order to remove any precipitated material. The heat-inactivated serum was then divided into aliquots and stored frozen at -20 °C until used within one month after preparation.

Commercially available sterile filtered heat-inactivated cynomolgus serum did not require further processing and was stored according to the supplier's recommendation.

### Modified saline solution

Two (2) mL of 0.1 N HCl and 4 mL of heat-inactivated monkey serum (prepared in Maccine or from a commercial supplier) was added to 194 mL of commercially available sterile saline. The pH of the solution was measured and adjusted to pH 4.0 using a dilute solution of HCl. The solution was sterile-filtered (0.22µm) before storage.

The final composition of this mixture was 0.001N HCl and 2% heat-inactivated monkey serum in saline. This can be used within 7 days when stored at 2-8 °C or for longer periods when stored at -20°C.

### Formulation of the Test Substance for Dosing

The required amount of test substance (without considering the peptide purity) was weighed and transferred into a suitable container. The drug was solubilized by adding the required amount of dimethyl sulfoxide (2% of the final formulation volume). The mixture was vortex-mixed or briefly sonicated until the drug was completely dissolved.

The modified saline solution was gradually added until the final volume was achieved (at this point, precipitates may form). The mixture was acidified with dilute hydrochloric acid until pH 4 to dissolve the precipitates and then sterile-filtered (0.22µm) prior to storage.

The dosing solutions was prepared weekly and, when not in use, stored at approximately 4 °C (2-8 °C) or at the appropriate storage condition based on the results of the stability analysis of PGL1001 in the vehicle formulation. On the days of dosing, the dosing solutions were equilibrated to room temperature and was dispensed at room temperature using syringes of appropriate sizes. No dilutions or filtrations were required. Actual amounts prepared were documented in the raw data.

### Necropsy and Post-mortem Evaluation

On the day of necropsy, blood samples were collected after an overnight fast and prior to sedation and euthanasia. The body weights were recorded prior to euthanasia. At the end of the study, animals were euthanized with an overdose of sodium pentobarbital under ketamine sedation followed by a standard necropsy. An external and internal examination was performed before the removal of any organs.

The pelvic cavity was exposed by midventral laparotomy. The reproductive tract tissues including the ovaries wer examined *in situ*. Any abnormalities were recorded with details of the location, colour, shape and size. The ovaries were photographed *in situ* before being carefully dissected free of surrounding tissue. Both ovaries were trimmed of the surrounding fat tissue, separated from the fallopian tubes, removed, measured, weighed, photographed, and immersed in 50 mL of Bouin's solution [75mL picric acid solution (1.3%), 20mL of formaldehyde, commercial solution (37%), and 5mL glacial acetic acid] for 24 hours and then transferred into 70% ethanol for histological processing. Carcasses were discarded following sampling of these tissues.

The fixed ovarian tissues were processed for paraffin embedding, slide preparation and staining. The fixed ovaries which were embedded in paraffin should be serially sectioned at 5 µm. Every tenth section were processed/ mounted onto a slide, stained with Papanicolaou trichrome and sent to the consultant for microscopic examination. The consultant was blinded as to the treatment given so no information on the treatment or group was included in the slide identifier.

The ovarian follicle count/analysis and histopathological evaluation was performed by the external consultant.

### Results:

In the cynomolgus monkeys, a 90 day treatment with Compound "A" at a low dose such as 0.4mg/kg triggered a reduction of the proportion of primordial follicles and an increase of the proportion of primary, secondary and pre-antral and antral follicles, when compared to ovarian follicles from the control animals. A 90 day treatment with Compound "A" at high dose such as 2.0mg/kg triggered a reduction of the proportion of atresia in the resting follicle population when compared to the control animals.

These results suggest that Compound "A" is relevant for accelerating the recruitment and the maturation of human ovarian follicle from the follicular reserve and for slowing down the depletion of the ovarian reserve.

At low dose, Compound "A" pharmacological effect is dominated by its antagonistic property resulting into a reduction of the proportion of primordial follicles when compared to ovarian follicles from the control animals.

At high dose, Compound "A" pharmacological effect is dominated by its agonistic property resulting into a slowdown of the apoptosis of the resting follicles and a protection of the ovarian reserve.

In this manner, the dual antagonistic/agonistic effect of Compound "A" manifests in distinct pharmacological effects that are beneficial in the treatment of infertility.

## Claims

1. Compound Cpa-[D-Cys-4-Pal-D-Trp-Lys-Thr-Cys]-2-Nal-NH₂ (Compound "A") and/or its non N-alkylated analogs, any pharmaceutically acceptable salt thereof or an active metabolite thereof for use to accelerate the start of growth of quiescent follicles and/or for use to slow down the depletion of the ovarian reserve.

2. Compound Cpa-[D-Cys-4-Pal-D-Trp-Lys-Thr-Cys]-2-Nal-NH₂ (Compound "A") and/or its non N-alkylated analogs, any pharmaceutically acceptable salt thereof or an active metabolite thereof, for use according to claim 1, in order to the treatment of infertility.

3. Compound Cpa-[D-Cys-4-Pal-D-Trp-Lys-Thr-Cys]-2-Nal-NH₂ (Compound "A") and/or its non N-alkylated analogs, any pharmaceutically acceptable salt thereof or an active metabolite thereof, for use according to claims 1 or 2, wherein said Compound "A", any pharmaceutically acceptable salt thereof or an active metabolite thereof is to be administered to a mammal, preferably to a human being.

4. Compound Cpa-[D-Cys-4-Pal-D-Trp-Lys-Thr-Cys]-2-Nal-NH₂ (Compound "A") and/or its non N-alkylated analogs, any pharmaceutically acceptable salt thereof or an active metabolite thereof, for use according to anyone of claims 1 to 3, wherein said human being is a woman of reproductive age, women undergoing Assisted Reproductive Treatment and preferably women undergoing Assisted Reproductive Treatment but are poor responders to FSH stimulation.

5. Compound Cpa-[D-Cys-4-Pal-D-Trp-Lys-Thr-Cys]-2-Nal-NH₂ (Compound "A") and/or its non N-alkylated analogs, any pharmaceutically acceptable salt thereof or an active metabolite thereof, for use according to anyone of claims 1 to 4, wherein said Compound "A" or any pharmaceutically acceptable salt thereof or a pharmaceutical formulation thereof is to be administered orally, or parenterally, preferably subcutaneously.

6. Compound Cpa-[D-Cys-4-Pal-D-Trp-Lys-Thr-Cys]-2-Nal-NH₂ (Compound "A") and/or its non N-alkylated analogs, any pharmaceutically acceptable salt thereof or an active metabolite thereof , for use according to anyone of claims 1 to 5, wherein said Compound Cpa-[D-Cys-4-Pal-D-Trp-Lys-Thr-Cys]-2-Nal-NH₂ (Compound "A") and/or its non N-alkylated analog, or any pharmaceutically acceptable salt thereof or an active metabolite thereof is administered daily, weekly or monthly.

7. Compound Cpa-[D-Cys-4-Pal-D-Trp-Lys-Thr-Cys]-2-Nal-NH₂ (Compound "A") and/or its non N-alkylated analogs, any pharmaceutically acceptable salt thereof or an active metabolite thereof , for use according to anyone of claims 1 to 6, wherein said Compound "A" and/or its non N-alkylated analog or any pharmaceutically acceptable salt thereof or an active metabolite thereof is administered in a daily dosage of 1 to 500 mg, preferably a daily dosage of 10mg to 200mg.

8. Compound Cpa-[D-Cys-4-Pal-D-Trp-Lys-Thr-Cys]-2-Nal-NH₂ (Compound "A") and/or its non N-alkylated analogs, any pharmaceutically acceptable salt thereof or an active metabolite thereof, for use according to claim 1, wherein the acceleration of the start of growth of quiescent follicles is for *in vitro* use in follicle cultures for the production of mature oocytes intended for fertilization.

9. Compound Cpa-[D-Cys-4-Pal-D-Trp-Lys-Thr-Cys]-2-Nal-NH₂ (Compound "A") and/or its non N-alkylated analogs, any pharmaceutically acceptable salt thereof or an active metabolite thereof, for use according to claim 1, wherein the acceleration of the start of growth of quiescent follicles is for use in toxicological analyses.

10. Pharmaceutical composition comprising Compound Cpa-[D-Cys-4-Pal-D-Trp-Lys-Thr-Cys]-2-Nal-NH₂ (Compound "A") and/or its non N-alkylated analogs, any pharmaceutically acceptable salt thereof or an active metabolite thereof, for use to accelerate the start of growth of quiescent follicles and/or for use to slow down the depletion of the ovarian reserve.

11. Pharmaceutical composition comprising Compound Cpa-[D-Cys-4-Pal-D-Trp-Lys-Thr-Cys]-2-Nal-NH₂ (Compound "A") and/or its non N-alkylated analogs, any pharmaceutically acceptable salt thereof or an active metabolite thereof, for use according to claim 10, in order to the treatment of infertility.

12. Pharmaceutical composition comprising Compound Cpa-[D-Cys-4-Pal-D-Trp-Lys-Thr-Cys]-2-Nal-NH₂ (Compound "A") and/or its non N-alkylated analogs, any pharmaceutically acceptable salt thereof or an active metabolite thereof, for use according to claims 10 or 11, wherein said Compound Cpa-[D-Cys-4-Pal-D-Trp-Lys-Thr-Cys]-2-Nal-NH₂ (Compound "A") and/or its non N-alkylated analog, any pharmaceutically acceptable salt thereof or an active metabolite thereof is to be administered to a mammal, preferably to a human being.

13. Pharmaceutical composition comprising Compound "A", any pharmaceutically acceptable salt thereof or an active metabolite thereof, for use according to anyone of claims 10 to 12, wherein said human being is a woman of reproductive age, women undergoing Assisted Reproductive Treatment and preferably women undergoing Assisted Reproductive Treatment but are poor responders to FSH stimulation.

14. Pharmaceutical composition comprising Compound Cpa-[D-Cys-4-Pal-D-Trp-Lys-Thr-Cys]-2-Nal-NH₂ (Compound "A") and/or its non N-alkylated analogs, any pharmaceutically acceptable salt thereof or an active metabolite thereof, for use according to anyone of claims 10 to 13, wherein said Compound Cpa-[D-Cys-4-Pal-D-Trp-Lys-Thr-Cys]-2-Nal-NH₂ (Compound "A") and/or its non N-alkylated analog, or any pharmaceutically acceptable salt thereof or a pharmaceutical formulation thereof is to be administered administered orally, or parenterally, preferably subcutaneously.

15. Pharmaceutical composition comprising Compound Cpa-[D-Cys-4-Pal-D-Trp-Lys-Thr-Cys]-2-Nal-NH₂ (Compound "A") and/or its non N-alkylated analogs, any pharmaceutically acceptable salt thereof or an active metabolite thereof, to anyone of claims 10 to 14, wherein said Compound Cpa-[D-Cys-4-Pal-D-Trp-Lys-Thr-Cys]-2-Nal-NH₂ (Compound "A") and/or its non N-alkylated analog or any pharmaceutically acceptable salt thereof or a pharmaceutical formulation thereof is administered daily, weekly or monthly.

16. Pharmaceutical composition comprising Compound Cpa-[D-Cys-4-Pal-D-Trp-Lys-Thr-Cys]-2-Nal-NH₂ (Compound "A") and/or its non N-alkylated analogs, any pharmaceutically acceptable salt thereof or an active metabolite thereof, to anyone of claims 10 to 15, wherein said Compound Cpa-[D-Cys-4-Pal-D-Trp-Lys-Thr-Cys]-2-Nal-NH₂ (Compound "A") and/or its non N-alkylated analog or any pharmaceutically acceptable salt thereof or a pharmaceutical formulation thereof is administered in a daily dosage of 1 to 500 mg, preferably a daily dosage of 10mg to 200mg.

17. Pharmaceutical composition comprising Compound Cpa-[D-Cys-4-Pal-D-Trp-Lys-Thr-Cys]-2-Nal-NH₂ (Compound "A") and/or its non N-alkylated analogs, any pharmaceutically acceptable salt thereof or an active metabolite thereof, for use according to claim 10, wherein the acceleration of the start of growth of quiescent follicles is for *in vitro* use in follicle cultures for the production of mature oocytes intended for fertilization..

18. Pharmaceutical composition comprising Compound Cpa-[D-Cys-4-Pal-D-Trp-Lys-Thr-Cys]-2-Nal-NH₂ (Compound "A") and/or its non N-alkylated analogs, any pharmaceutically acceptable salt thereof or an active metabolite thereof, for use according to claim 10, wherein the acceleration of the start of growth of quiescent follicles is for use in toxicological analyses.

19. A pharmaceutical pack or kit comprising one or more containers filled with Compound Cpa-[D-Cys-4-Pal-D-Trp-Lys-Thr-Cys]-2-Nal-NH₂ (Compound "A") and/or its non N-alkylated analogs, any pharmaceutically acceptable salt thereof or an active metabolite thereof.

20. A method for the acceleration the start of growth of quiescent follicles and/or for slowing down the depletion of the ovarian reserve comprising administering Compound Cpa-[D-Cys-4-Pal-D-Trp-Lys-Thr-Cys]-2-Nal-NH₂ (Compound "A") and/or its non N-alkylated analogs, any pharmaceutically acceptable salt thereof or an active metabolite thereof.

21. The method according to claim 18, for the treatment of infertility.

22. The method according to claims 18 or 19, wherein said Compound Cpa-[D-Cys-4-Pal-D-Trp-Lys-Thr-Cys]-2-Nal-NH₂ (Compound "A") and/or its non N-alkylated analog, any pharmaceutically acceptable salt thereof or an active metabolite thereof is to be administered to a mammal, preferably to a human being.

23. The method according to anyone of claims 20 to 22, wherein said human being is woman of reproductive age, women undergoing Assisted Reproductive Treatment and preferably women undergoing Assisted Reproductive Treatment but are poor responders to FSH stimulation.

24. The method according to anyone of claims 20 to 23, wherein said Compound Cpa-[D-Cys-4-Pal-D-Trp-Lys-Thr-Cys]-2-Nal-NH₂ (Compound "A") and/or its non N-alkylated analog, any pharmaceutically acceptable salt thereof or an active metabolite thereof is to be administered orally, or parenterally, preferably subcutaneously.

25. The method according to anyone of claims 20 to 24, wherein said Compound Cpa-[D-Cys-4-Pal-D-Trp-Lys-Thr-Cys]-2-Nal-NH₂ (Compound "A") and/or its non N-alkylated analog, any pharmaceutically acceptable salt thereof or an active metabolite thereof is to be administered daily, weekly or monthly.

26. The method according to anyone of claims 20 to 25, wherein said Compound Cpa-[D-Cys-4-Pal-D-Trp-Lys-Thr-Cys]-2-Nal-NH₂ (Compound "A") and/or its non N-alkylated analog, any pharmaceutically acceptable salt thereof or an active metabolite thereof is to be administered in a daily dosage of 1 to 500 mg, preferably a daily dosage of 10mg to 200mg.

27. The method according to claim 20, wherein the acceleration of the start of growth of quiescent follicles is for *in vitro* use in follicle cultures for the production of mature oocytes intended for fertilization.

28. The method according to claim 20, wherein the acceleration of the start of growth of quiescent follicles is for use in toxicological analyses.
